Europäisches Patentamt

European Patent Office

Office européen des brevets

(11) Veröffentlichungsnummer: **0 090 997**
**B1**

## EUROPÄISCHE PATENTSCHRIFT

(45) Veröffentlichungstag der Patentschrift:
**18.10.89**

(21) Anmeldenummer: **83102773.5**

(22) Anmeldetag: **21.03.83**

(51) Int. Cl.⁴: **A 61 L 15/04, A 61 L 15/03**

(54) Gewebeverklebbare kollagene Wundauflage.

(30) Priorität: **02.04.82 DE 3212412**

(43) Veröffentlichungstag der Anmeldung:
**12.10.83 Patentblatt 83/41**

(45) Bekanntmachung des Hinweises auf die Patenterteilung:
**18.10.89 Patentblatt 89/42**

(84) Benannte Vertragsstaaten:
**AT BE CH DE FR GB IT LI LU NL SE**

(56) Entgegenhaltungen:
**EP-A- 0 049 469**
**EP-A- 0 059 265**
**EP-A- 0 068 047**
**FR-A- 2 422 407**

**CHEMICAL ABSTRACTS, Band 94, Nr. 2, 12. Januar
1981, Seite 246, Nr. 7649t, Columbus, Ohio, US; A.
STEMBERGER u.a.: "Biochemical and physiological
aspects of fibrin adhesion" & FIBRINOGEN, FIBRIN
FIBRINKLEBER, VERHANDLUNGSBER. DTSCH.
ARBEITSGEM. BLUTGERINNUNGSFORSCH. TAG., 23rd
1979 (Pub. 1980), 209-16**

(73) Patentinhaber: **Dr. Ruhland Nachf. GmbH, Stadtplatz 7,
D-8425 Neustadt/Donau (DE)**

(72) Erfinder: **Stemberger, Axel, Dr.-rer.nat.,
Cramer-Klett-Strasse 35e, D-8014 Neublberg (DE)**

(74) Vertreter: **Hansen, Bernd, Dr.rer.nat. et al, Hoffmann,
Eitle & Partner Patentanwälte
Arabellastrasse 4 Postfach 81 04 20,
D-8000 München 81 (DE)**

ACTORUM AG

### Beschreibung

Kollagen wird seit geraumer Zeit in der Chirurgie verwendet. Es kann in Form von Schwämmen oder Fasern zur Blutstillung verwendet werden und ist auch nach entsprechender Modifizierung zur Beschleunigung der Wundheilung geeignet. Bei Patienten mit defekter Blutgerinnung oder bei großflächigen Blutungen genügen übliche kollagene Wundauflagen nicht. Man hat daher bereits versucht, Kollagenmaterial mit dem Gewebe zu verkleben, wobei man Kleber auf Basis Resorcin-Formaldehyd, Kollagen oder Gelatine verwendete. Solche Kleber sind zwar hämostyptisch, jedoch wegen ihrer Toxizität für die Praxis ungeeignet. Dies gilt auch für Acrylatkleber und die Kombination mit kollagenen Wundauflagen.

Es ist bekannt, daß Kollagen kovalente Vernetzungen mit Bindegewebebestandteilen eingehen kann. Dabei finden Vernetzungen vom Kollagen über Schiff-Basen sowie Aldolkondensation statt. Bekannt ist auch, daß bei Basalmembranen die Gewebefestigkeit zusätzlich durch S–S Brücken des Basalmembrankollagens erreicht wird. Bekannt ist weiterhin, Proteine, wie Albumin mit intermolekularen S–S Bindungen nach milder Reduktion und nachfolgender Oxidation über S–S Brücken zu vernetzen.

Bei Verletzungen bildet sich durch die Blutgerinnung ein primärer Wundverschluß. Hierfür sind aggregierte Thrombozyten und ein Fibrinnetzwerk verantwortlich. Es ist bekannt, daß einzelne Fibrinmoleküle durch Transglutaminasen (wie Faktor XIII) vernetzt werden. Hierbei bilden sich neue Peptidbindungen zwischen Glutaminsäure und Lysin benachbarter Ketten. Mit der Technik der Fibrinklebung, also durch Verwendung von Fibrinogen und Thrombin, wird die Endphase der plasmatischen Blutgerinnung nachgeahmt.

Die Fibrinklebung allein ist nicht in der Lage, großflächige Blutungen zu stillen. Dies gelingt erst durch eine kombinierte Anwendung der Fibrinklebung mit einer resorbierbaren kollagenen Wundauflage. Jedoch muß man dabei drei Komponenten bereithalten, nämlich die kollagene Wundauflage, Thrombinlösung mit Antifibrinolytika und eine tiefgefrorene hochkonzentrierte Fibrinogenlösung. Da Blutungen häufig plötzlich und unprogrammiert auftreten, stehen diese drei Komponenten im entscheidenden Augenblick häufig nicht anwendungsbereit zur Verfügung, denn zumindest die tiefgefrorene Fibrinogenlösung muß zuvor aufgetaut werden. Auch das Mischen vor der Applikation ist verhältnismäßig kompliziert.

In der «Wiener medizinische Wochenschrift Nr. 7 (1976)» wird auf den Seiten 86 bis 89 über die lokale Anwendung von Fibrinogen und Kollagen zur Blutstillung in der Herzchirurgie berichtet. Bei der Technik der dort beschriebenen Klebung verwendet man humanes Fibrinogen-Kryopräzipitat, Thrombin, ein Faktor XIII Konzentrat sowie ein Kollagenvlies. Das Fibrinogen wird auf die gewünschte Stelle aufgebracht und dort durch den Zusatz der Thrombinlösung und von Faktor XIII zur Gerinnung gebracht. Bei stärkeren Blutungen wendet man das Kollagenvlies als vollständig resorbierbare Trägersubstanz an, wobei man auf das Kollagenvlies die Lösungen aufträgt und anschließend das Kollagen mit der mit Fibrinogen beschichteten Seite auf die blutende Stelle aufpreßt. Dadurch soll eine vollständige Auspolymerisierung des Fibrins und Haftung der Fibrinfasern im Gewebe erfolgen. Bei dieser Klebetechnik erfolgt die Beschichtung des Kollagens mit Fibrinogen erst unmittelbar vor der Anwendung, um ein Kollabieren des Kollagenschwammes weitgehend zu vermeiden.

Aufgabe der Erfindung ist es, dem Chirurgen eine kollagene Wundauflage zur Verfügung zu stellen, die in vorbereiteter Form direkt während der Operation angewendet werden kann und wobei die vorerwähnten Nachteile der Fibrinklebung in Kombination mit resorbierbarem Kollagen nicht auftreten. Verbunden mit dieser Aufgabe ist es, eine lagerfähige Kollagenzubereitung zur Verfügung zu stellen, die die für die Einleitung der lokalen Hämostase erforderlichen Komponenten enthält.

Diese Aufgabe wird durch eine gewebeverklebbare, kollagene Wundauflage gemäß dem Anspruch 1 gelöst.

Die erfindungsgemäße Wundauflage hat einen schichtweisen Aufbau. Die Hauptschicht besteht hierbei aus dem Kollagen und zwar hat diese Schicht eine Dicke zwischen etwa 0,3 und 2 cm, vorzugsweise 0,7 bis 1 cm. Einseitig oder beidseitig auf dieser Kollagenschicht befindet sich eine Fibrinogenschicht. Diese Fibrinogenschicht hat eine Dicke von etwa 0,2 bis 2 mm und wird auf die Kollagenoberfläche aufgebracht, wobei eine Verankerung der Fibrinogenmoleküle mit den Kollagenmolekülen im Grenzgebiet der Schichten vorliegt.

Wesentlich ist, daß die Fibrinogenschicht, Fibrinogen in einer Menge von 0,5 bis 10 mg, vorzugsweise 4 mg/cm$^2$ enthält.

Es ist häufig vorteilhaft, wenn das Fibrinogen das einseitig oder beidseitig auf das Kollagen als Schicht aufgebracht wird, freie SH-Gruppen enthält. Diese freien SH-Gruppen können zuvor in dem Fibrinogen durch reduktive Spaltung der Disulfidbrücken ausgebildet sein, oder indem man Fibrinogen mit SH-Gruppen haltigen Fibrinogen abmischt. Weiterhin ist es auch möglich die in Fibrinogen enthaltenen Disulfidbrücken unverändert zu lassen und zusätzlich SH-Gruppen einzubauen, z.B. nach der Methode von Benesch & Benesch in «Proceedings of the National Academy of the USA, Washington», Band 44, 1958, S. 848 bis 853 oder Biochem. Journ. 1966 101, Seiten 717 bis 720 (Stephen und Mitarbeiter).

Es kann vorteilhaft sein, wenn neben dem Fibrinogen wenigstens eine der Schichten noch Antifibrinolytika und/oder polyvalente Proteinaseninhibitoren enthält, wobei außerdem noch Thrombin vorhanden sein kann, mit dem Proviso, daß das Thrombin sich nicht in der gleichen Schicht oder in unmittelbarem Kontakt mit Fibrinogen befindet.

Die Herstellung der erfindungsgemäßen kollagenen Wundauflage kann in folgender Weise erfolgen:

Zunächst wird Kollagen in an sich bekannter Weise präpariert. Das Kollagen soll dabei vorzugsweise einen Reinheitsgrad, ausgedrückt durch das Gewichtsverhältnis von Stickstoff zu Hydroxyprolin <4, insbesondere <3 aufweisen.

$$\text{Faktor N/Hyp} = \frac{\text{mg N}}{\text{mg 4-Hyp}}$$

J. Mol. Bio. 44, 1969, Seite 161.

Eine 1,5%-ige Kollagenlösung in 0,05%-iger Essigsäure wird in bekannter Weise zu einem 0,5 cm dicken Kollagenschwamm gefriergetrocknet. Erwünschtenfalls kann man bei dieser Gefriertrocknung auch eine Lösung einsetzen, welcher man zuvor Antifibrinolytika (Tranexamsäure) und/oder poyvalente Proteinaseninhibitoren (Aprotinin) zugegeben hat, wobei diese in solchen Mengen zugegeben werden, daß im fertigen Kollagenschwamm pro cm² eine Konzentration von 0,2 bis 10 mg bzw. 4- bis 1000 KIE vorliegt.

Getrennt von der Herstellung des Kollagenschwamms wird eine Fibrinogenlösung wie folgt hergestellt:

Fibrinogen für Infusionszwecke wird in isot. NaCl bis zu einer Konzentration von 30 mg Fibrinogen pro ml gelöst. Diese Lösung wird dann mit einer üblichen Sprühvorrichtung auf den zuvor hergestellten Kollagenschwamm unter sterilen Kautelen in einer Dicke von 0,2 bis 2 mm aufgesprüht, die eine Menge von 0,5 bis 10 mg/cm² ergibt, sofort gefriergetrocknet, wobei die dadurch ausgebildete Schichtdicke in der Regel 2 mm Schichthöhe nicht übersteigen darf, weil sonst die Haftung dieser Schicht schlecht ist. Mit dieser Technik wird eine ausreichende Verankerung in der Kollagenmatrix sichergestellt. Gewünschtenfalls können auch in die Fibrinogenschicht Antifibrinolytika und/oder polyvalente Proteinaseninhibitoren eingearbeitet werden.

Es ist bekannt, Kollagen als Träger für Antibiotika, wie Gentamycin zu verwenden. Auch die erfindungsgemäßen Wundauflagen können Wirkstoffe enthalten, beispielsweise Gentamycin, Tetracyclin oder andere Antibiotika oder Chemoterapeutika.

Herstellung von Kollagen

Von allen Pigmentschichten und Muskelresten befreite frische Rindersehnen wurden homogenisiert und eine Menge, die 100 g Trockengewicht entspricht, in 3 l 0,05 m Zitratpuffer (pH 3,7) 24 Stunden lang extrahiert und anschließend gegen 1% Essigsäure 12 Stunden dialysiert.

Das in 3 l 1% Essigsäure suspendierte Gewebe wird 48 Stunden bei 10°C unter ständigem Rühren mit Pepsin im Verhältnis Kollagen : Pepsin = 50 : 1 inkubiert.

Der Ansatz wird mit 1% Essigsäure auf 5 l verdünnt und durch Zentrifugation von den ungelösten Sehnenfragmenten befreit.

Die viskose Kollagenlösung wird gegen alkalisiertes Leitungswasser (pH 8,0) dialysiert und dann scharf zentrifugiert. Der Rückstand wird erneut in 5 l 1% Essigsäure gelöst und dialysiert. Dieser Vorgang wird wiederholt, bis der Faktor Stickstoff:4-Hydroxyprolin <3 ist. Nach dem letzten Dialysieren wird mit 0,05% Essigsäure eine 1,5%-ige Kollagenlösung hergestellt, die für die nachfolgenden Versuche verwendet wird.

Zur Herstellung einer Antifibrinolytika- und/oder Proteinaseninhibitor-haltigen Kollagenschwammes von 10 × 10 × 0,5 cm werden 50 ml der Kollagenlösung mit 0,2 g Tranexamsäure und/oder 40.000 KIE Aprotinin versetzt und gefriergetrocknet.

Zur Herstellung eines Kollagen-Gentamycin-Schwamms von 10 × 10 × 0,5 cm werden 50 ml der Kollagenlösung auf pH 1 bis 2 eingestellt, mit 100 mg Gentamycinsulfat versetzt und gefriergetrocknet.

Herstellung von Fibrinogenlösung

Im Handel befindliches steriles Fibrinogen wird in steriler isotoner Na HCl gelöst, so daß eine Lösung von 30 mg Fibrinogen/ml Lösung vorliegt, die für die nachfolgenden Versuche verwendet wird.

Um ein SH-Gruppen-modifiziertes Fibrinogen zu erhalten, arbeitet man wie folgt:

Fibrinogen wird in isotoner Kochsalzlösung bis zu einer Konzentration von 20 mg/ml gelöst. 10 ml dieser Fibrinogenlösung werden mit 1 ml einer N-Acetylhomocysteinthiolaktonlösung (60 mg/ml Agua dest) und 10 ml eines Carbonatpuffers (pH 10,6) versetzt und bei 0°C 35 Minuten inkubiert. Die Reaktion wird dann durch Zugabe von 40 ml eines Phosphatpuffers pH 7,0 i 0,4 gestoppt. Anschließend wird mit der Technik der Membranfiltration unter Verwendung eines Filters (Ausschlußgrenze Molekulargewicht 5.000) die Lösung entsalzt und gleichzeitig eingeengt.

Herstellung von Wundauflagen, die Kollagen und Fibrinogen enthalten

Es wird zunächst 100 ml einer 1%-igen Kollagenlösung in eine Metallform von 10 × 10 cm gegeben, nach üblicher Technik gefriergetrocknet und der resultierende Schwamm dann sterilisiert. Unter aseptischen Bedingungen wird nun dieser sterilisierte Kollagenschwamm mit einer Fibrinogenlösung besprüht, wobei pro Quadratzentimeter Kollagenoberfläche 5 mg Fibrinogen aufgetragen werden.

Anschließend erfolgt eine erneute Gefriertrocknung und Verpackung unter sterilen Kautelen.

Die Kollagenschicht hat eine Dicke von 10 mm und die darauf befindliche Fibrinogenschicht eine Dicke von ca. 0,3 mm.

Resultate der in vitro und in vivo Versuche mit Kollagen-Fibrinogen- sowie Gentamycin-Kollagen-Fibrinogen-Schwämmen.

In vitro

Die so hergestellten Wundauflagen wurden mit einer Reißfestigkeitsmaschine getestet, bestehend aus einem Kraftaufnehmer, verbunden mit einem Schreiber. Die Eichung erfolgte im Bereich von 100 bis 1000 Pond. Die Kollagen-Fibrinogenschwämme wurden mit der Kollagenseite auf eine Plastikscheibe (D = 1 cm) mittels eines technischen Klebers geklebt. Die fibrinogenbeschichtete Seite wurde dann mit 0,1 ml Thrombinlösung (1000 NIH/ml) befeuchtet und nach 20 Minuten in der Testmaschine die Reißfestigkeit bestimmt. Es resultierten Werte von 730 Pond/cm² (Mittelwert aus 7 Messungen).

In vivo

In einer Studie an 400 Ratten wurden die (Gentamycin) Kollagen-Fibrinogen-Wundauflagen (als Fibrocoll bezeichnet) zur Anastomosensicherung am Colon mit der herkömmlichen Gewebeklebung mit Plasmafraktionen (Fibrinklebesystem = FK) untersucht.

a) Kontrollgruppe (Enterotomien mit 7 invertierenden Einzelknopfnähten nach Lembert.

b) zusätzliche Applikation von FK

c) FK mit Kollagenvlies (fibrinogen-, gentamycin-frei)

d) Nahtsicherung mit Fibrocoll

e) Fibrocoll mit Gentamycin (1 mg/cm²)

In der Initialphase der Wundheilung zeigten die mit verschiedenen Klebesystemen behandelten Tiere bis zum 3. postoperativen Tage im Nahtbereich eine deutlich höhere Wundfestigkeit. Gegenüber der herkömmlichen Fibrinklebetechnik waren bei Verwendung von Fibrocoll Adhäsionen im Wundbereich vermindert. Durch die Applikation der Gentamycin-Kollagen-Fibrinogen Wundauflagen wurde eine kontrollierte Freigabe des Antibiotikums beobachtet, die Serumkonzentrationen lagen weit unter der toxischen Grenze.

In einer weiteren Versuchsserie wurden Hemihepatektomien in «finger fracture technigne» bei 5 Schweinen durchgeführt und nach Ligatur der faßbaren Arterien und Venen die blutenden Parenchymdefekte mit Kollagen-Fibrinogen-Wundauflagen versorgt. Im folgenden postoperativem Verlauf wurden Nachblutungen nie beobachtet, die Resorption der Materialien war nach 12 Wochen abgeschlossen.

Mit den (Gentamycin) Kollagen-Fibrinogen-Wundauflagen steht ein Material zur Verfügung das bei einfacher, rascher, sicherer Handhabung ausgezeichnet zur Blutstillung eingesetzt werden kann. Weiter wird eine Verbesserung der Wundheilung bei gleichzeitigem Antibiotikaschutz beobachtet.

**Patentansprüche**

1. Trockene, vlies- oder schwammförmige, gewebeverklebbare, flächenförmige, kollagene Wundauflage aus Kollagen und Fibrinogen, dadurch gekennzeichnet, daß sie schichtweise durch Gefriertrocknung einer Kollagen/Fibrinogen-Kombination aufgebaut ist und eine 0,3 bis 2 cm dicke Kollagenschicht enthält, die wenigstens an einer Oberfläche eine 0,2 bis 2 mm dicke Fibrinogenschicht mit einer Fibrinogenmenge von 0,5 bis 10 mg/cm² aufweist und in dem Kollagen verankert ist.

2. Wundauflage gemäß Anspruch 1, dadurch gekennzeichnet, daß das Fibrinogen SH-Gruppen enthält.

3. Wundauflage gemäß Anspruch 2, dadurch gekennzeichnet, daß das Fibrinogen SH-Gruppen enthält, die entweder durch nachträgliche Sulfhydrierung in das Fibrinogenmolekül eingebracht oder durch Reduktion der Disulfidbrücken im Fibrinogenmolekül gebildet wurden.

4. Wundauflage gemäß einem der vorhergehenden Ansprüche, dadurch gekennzeichnet, daß sie zusätzlich einen Arzneimittelwirkstoff enthält.

5. Wundauflage gemäß Anspruch 4, dadurch gekennzeichnet, daß sie in wenigstens einer der Schichten Antifibrinolytika und/oder polyvalente Proteaseninhibitoren und/oder Thrombin enthält, wobei jedoch die Kombination Thrombin-Fibrinogen in einer Schicht ausgeschlossen ist.

6. Wundauflage gemäß Anspruch 4, dadurch gekennzeichnet, daß der Arzneimittelwirkstoff ein Antibiotikum ist.

7. Wundauflage gemäß Anspruch 6, dadurch gekennzeichnet, daß das Antibiotikum Gentamycin ist.

8. Wundauflage gemäß Anspruch 1, dadurch gekennzeichnet, daß das Kollagen einen Reinheitsgrad, ausgedrückt durch das Gewichtsverhältnis von Stickstoff zu 4-Hydroxyprolin von < 4, vorzugsweise < 3 hat.

**Claims**

1. Dry, Fleecy or spongy tissue-adherent flat collagenous dressing made of collagen and fibrinogen, characterized in that the dressing is made in layers by freeze-drying a collagen/fibrinogen combination and contains a collagen layer which is 0.3 to 2 cm thick, and which has on at least one surface a fibrinogen layer which is 0.2 to 2 mm thick with a fibrinogen content of from 0.5 to 10 mg/cm² and is anchored in the collagen.

2. Dressing according to claim 1, characterized in that dibrinogen contains SH groups.

3. Dressing according to claim 2, characterized in that the fibrinogen contains SH groups which either are introduced into the fibrinogen molecule by subsequent sulfhydrogenation or are formed by reduction of the disulfide bridges in the fibrinogen molecule.

4. Dressing according to any one of the preceding claims characterized in that it additionally contains a pharmaceutical.

5. Dressing according to claim 4, characterized in that it contains in at least one of the layers antifibrinolytics and/or polyvalent proteinase inhibitors and/or thrombin, while however the combination thrombin-fibrinogen is excluded in one layer.

6. Dressing according to claim 4, characterized in that the pharmaceutical is antibiotic.

7. Dressing according to claim 6, characterized in that the antibiotic is gentamycin.

8. Dressing according to claim 1, characterized in that the collagen has a degree of purity expressed as the weight ratio of nitrogen to 4-hydroxyproline of less than 4, preferably less than 3.

**Revendications**

1. Matériau de recouvrement des plaies à base de collagène, adhésif plat et sec, en forme de nappe ou d'éponge, caractérisé en ce qu'il est constitué en couches par cryodessication d'une combinaison de collagène et de fibrinogène et qu'il contient une couche de collagène d'une épaisseur de 0,3 à 2 cm, qui présente au moins sur une surface une couche de fibrinogène d'une d'épaisseur de 0,2 à 2 mm, avec une quantité de fibrinogène comprise entre 0,5 et 10 mg/cm² et qui est ancrée dans le collagène.

2. Matériau de recouvrement selon la revendication 1, caractérisé en ce que le fibrinogène contient des groupes SH.

3. Matériau de recouvrement selon la revendication 2, caractérisé en ce que le fibrinogène contient des groupes SH, soit introduits dans la molécule de fibrinogène par sulfhydrogénation ultérieure, soit formés par réduction des ponts disulfides dans la molécule de fibrinogène.

4. Matériau de recouvrement selon l'une des revendications précédentes, caractérisé en ce qu'il contient en plus une substance médicamenteuse.

5. Matériau de recouvrement selon la revendication 4, caractérisé en ce qu'il contient dans au moins l'une des couches un antifibrinolytique et/ou des inhibiteurs de protéinase équivalents et/ou une thrombine, la combinaison thrombine-fibrinogène étant placée dans une couche séparée.

6. Matériau de recouvrement selon la revendication 4, caractérisé en ce que la substance médicamenteuse est un antibiotique.

7. Matériau de recouvrement selon la revendication 6, caractérisé en ce que l'antibiotique est la gentamycine.

8. Matériau de recouvrement selon la revendication 1, caractérisé en ce que le collagène présente un degré de pureté, exprimé par le rapport de poids entre azote et 4-hydroxyproline, <4, de préférence <3.